# EUROPEAN PATENT SPECIFICATION

(11) **EP 3 785 667 B1**
(45) Date of publication and mention of the grant of the patent: **28.06.2023**
(21) Application number: 20153062.3
(22) Date of filing: 17.03.2016
(51) Int. Cl.: A61F 2/08

(54) **HIGH-STRENGTH ALLOGRAFT TENDON CONSTRUCT**
HOCHFESTES ALLOGENES SEHNENKONSTRUKT
CONSTRUCTION DE TENDON D'ALLOGREFFE HAUTE RÉSISTANCE

(43) Date of publication of application: 03.03.2021
(62) Divisional of application: 16894757.0
(73) Proprietor: AlloSource, Centennial, CO 80111 (US)
(72) Inventor: Samaniego, Adrian, Centennial, Colorado 80111 (US); Southard, Matt, Centennial, Colorado 80111 (US); Franklin, Wendy Desiree, Centennial, Colorado 80111 (US); Hodgkiss, Jessica, Centennial, Colorado 80111 (US); Karaze, Bilal, Centennial, Colorado 80111 (US)
(74) Representative: Loo, Chi Ching

(56) References cited:
- US-A1- 2007 239 275
- US-A1- 2008 027 485
- US-A1- 2012 253 465

## Description

### Background

An allograft includes bone, tendon, skin, or other types of tissue transplanted from one person to another. Allografts are used in a variety of medical treatments, such as knee replacements, bone grafts, spinal fusions, eye surgery, and skin grafts for the severely burned. Allografts come from voluntarily donated human tissue obtained from donor-derived, living-related, or living-unrelated donors and can help patients regain mobility, restore function, enjoy a better quality of life, and even save lives.

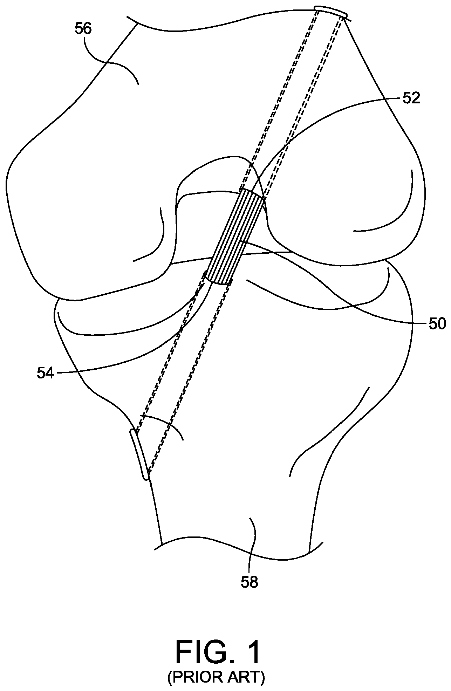

When a ligament or tendon becomes detached from the bone, surgery is usually required to re-secure the ligament or tendon. Often, a substitute allograft ligament or tendon (hereinafter an "allograft construct" or "allograft tendon construct") is attached to the bone to facilitate regrowth and permanent attachment. The reattachment procedure involves drilling a bone tunnel between two bones such as, for example, the tibia and the femur, and securing the allograft construct within the tunnel. To demonstrate this technique, Prior Art FIG. 1 shows an exemplary prior art allograft construct 50 secured within femoral and tibial tunnels 52, 54, each formed in femur and tibia 56, 58, respectively.

An allograft construct must be properly tensioned within the bone tunnel to achieve optimal results. That is, the tension or the "fit" of the allograft construct within the bone tunnel prior to being anchored to the bone must be sufficient to achieve stability, but not so excessive that it captures the joint. One variable in achieving optimal tension of the allograft construct within the bone tunnel involves preparing an allograft construct having the proper cross-sectional diameter. Preparing a construct with the requisite cross-sectional diameter typically involves folding a single tendon strand in half, which results two abutting tendon lengths having in a common middle region bounded by a folded end and a free end. The free end may then be whip stitched together. Alternatively, two separate tendon strands may be associated with one another, or "doubled up," before one or both free ends are whip stitched together.

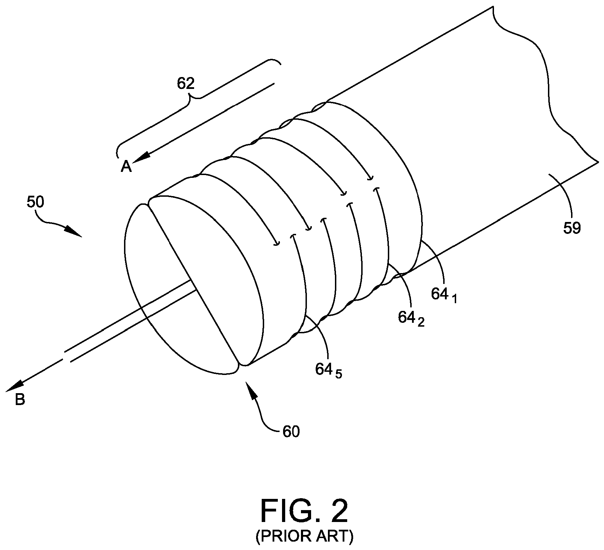

Prior Art FIG. 2 illustrates a partial perspective view of an unstitched middle region 59 and a free end 60 of prior art allograft construct 50, in which free end 60 has been whip stitched using a flexible strand to form a stitched pattern 62. Notably, the whip stitched pattern 62 of prior art allograft construct 50 originates inward toward unstitched middle region 59, from where a number of sutures 64₁₋₅ progress or advance outward toward free end 60 along arrow A. As a result, final suture 64₅ is located adjacent to free end 60, and pulling forces applied to the flexible strand along arrow B are transferred to final suture 64₅ at free end 60.

Prior art stitch pattern 62 is often applied to a folded allograft tendon, and discussed above. Alternatively, it is applied to two independent tendon strands that are stitched together at one or both of their ends using a similar whip stitching technique.

Traditionally, surgeons have been responsible for tendon graft preparation, individually preparing appropriately cross-sectioned, whip stitched grafts for each patient and/or circumstance. Recently, pre-sutured allograft constructs have become available from third-part providers, such as, for example, allograft processing centers, thereby allowing surgeons to order high quality, consistent, strong, and sterile tendon allografts, either individually or as part of a larger "kit" carrying a variety of sizes. US2012/253465A1 discloses a method for preparing a tendon bundle as a replacement ligament or tendon. US2007/239275A1 discloses a graft construct formed of a plurality of single tendon strands or soft tissue grafts. US2008/027485A1 discloses a system and method for tissue repair employing a continuous loop of suture attached to a free floating needle.

### Summary

This Summary is provided to introduce a selection of concepts in a simplified form that are further described below in the Detailed Description. This Summary is not intended to identify key aspects or essential aspects of the claimed subject matter. Moreover, this Summary is not intended for use as an aid in determining the scope of the claimed subject matter.

In accordance with the present invention there is provided a non-surgical method of tendon reconstruction. The method includes (1) positioning a first tendon length and a second tendon length longitudinally in parallel to one another; (2) threading a flexible strand through and about a first free end of the first and second tendon lengths to form an allograft construct having a stitched end portion that abuts an unstitched middle portion, where the threading comprises forming a plurality of sutures by advancing the flexible strand away from a first suture located adjacent to the first free end and toward a final suture located adjacent to the unstitched middle portion; and (3) after forming the final suture, stringing the flexible strand through a center space between the first and second tendon lengths and out the first free end of the stitched end portion.

In accordance with the present invention there is also provided an allograft construct as claimed in claim 1.

Another embodiment not in accordance with the present invention provides a substitute tendon having at least a stitched end portion and an unstitched middle portion and including a continuous flexible strand forming a whip stitched pattern and an anchor. The whip stitched pattern comprises a plurality of sutures that secure a common free end of two longitudinally abutting tendon lengths, the plurality of sutures originating with a first suture located adjacent to the free end and progressing toward a final suture located adjacent to the unstitched middle portion. The anchor originates at the final suture and threads through a space between the tendon lengths and out the common free end of the stitched end portion.

Other embodiments are also disclosed.

Additional objects, advantages and novel features of the technology will be set forth in part in the description which follows, and in part will become more apparent to those skilled in the art upon examination of the following, or may be learned from practice of the technology.

### Brief Description of the Drawings

Non-limiting and non-exhaustive embodiments of the present invention, including the preferred embodiment, are described with reference to the following figures, wherein like reference numerals refer to like parts throughout the various views unless otherwise specified. Illustrative embodiments of the invention are illustrated in the drawings, in which:
FIGURE 1 illustrates a perspective view of a prior art allograft construct affixed within femoral and tibial bone tunnels of a patient;
FIGURE 2 illustrates a partial-perspective view of the prior art allograft construct of FIGURE 1;
FIGURE 3 illustrates a partial-perspective view of one embodiment of a high-strength, pre-sutured allograft tendon construct;
FIGURES 4A-4C illustrate respective partial-side, end, and partial-perspective views of first and second tendon lengths of the allograft construct of FIGURE 3;
FIGURE 4D illustrates a perspective view of one embodiment of a folded-over tendon strand forming the first and second tendon lengths of FIGURES 4A-4C;
FIGURES 5A-5C illustrate respective partial-side, end, and partial-perspective views of the first and second tendon lengths of FIGURES 4A-4C, including a first suture applied adjacent to a free end;
FIGURES 6A-6C illustrate respective partial-side, end, and partial-perspective views of the first and second tendon lengths of FIGURES 4A-4C, including a first suture located adjacent to a free end and a second suture applied in a direction progressing inward toward an unstitched middle portion;
FIGURES 7A-7C illustrate respective partial-side, end, and partial-perspective views of the first and second tendon lengths of FIGURES 4A-4C, including first through fourth sutures that originate adjacent to a free end and progress inward toward an unstitched middle portion;
FIGURES 8A-8C illustrate respective partial-side, end, and partial-perspective views of the first and second tendon lengths and sutures of FIGURES 7A-7C, including a Duncan Loop knot applied after the fourth suture;
FIGURE 9 illustrates a partial-side view of the first and second tendon lengths, sutures, and Duncan Loop knot of FIGURES 8A-8C, including three Reverse-Half-Hitch-Alternating-Post knots applied after the Duncan Loop knot;
FIGURE 10 illustrates a partial-side view of the first and second tendon lengths, sutures, and knots of FIGURE 9, where the knots are tightened into a multiple knot bundle;
FIGURES 11A-11C illustrate respective partial-side, end, and partial-perspective views of the first and second tendon lengths, sutures, and multiple knot bundle of FIGURE 10, including an initiated fifth suture;
FIGURES 12A-12C illustrate respective partial-side, end, and partial-perspective views of the first and second tendon lengths, sutures, and multiple knot bundle of FIGURES 11A-11C, with an anchor threaded through a space between the tendon lengths and out a free end;
FIGURE 13 illustrates a partial-perspective view of the high-strength, pre-sutured allograft tendon construct of FIGURE 3, including indications of a direction of suture progression and directions of force application and force transfer; and
FIGURE 14 illustrates a flow chart depicting an exemplary method of manufacturing the high-strength, pre-sutured allograft tendon construct of FIGURE 13.

### Detailed Description

Embodiments are described more fully below in sufficient detail to enable those skilled in the art to practice the system and method. However, embodiments may be implemented in many different forms and should not be construed as being limited to the embodiments set forth herein. The following detailed description is, therefore, not to be taken in a limiting sense.

Various embodiments of the systems and methods described herein relate to a pre-sutured, high-strength allograft construct that may be pre-ordered and made available for a surgeon to position and affix in place within a target bone tunnel(s) of a patient. The allograft construct features a whip stitched suture pattern that outperforms current methods of suturing tendons and ligaments for use in tendon replacement surgeries (e.g., ACL replacement surgeries). One embodiment provides an allograft tendon construct having a whip stitched pattern that originates at an outermost end of the allograft construct and progresses inward. The new suture pattern results in a stronger final product that increases the maximum force that can be applied to a pre-sutured tendon construct without risking suture pull-out and/or slippage. This unique pattern also prevents deformation of the allograft tendon construct when subjected to the type of excessive tensile forces that are often applied to the suture pattern after surgery and in use.

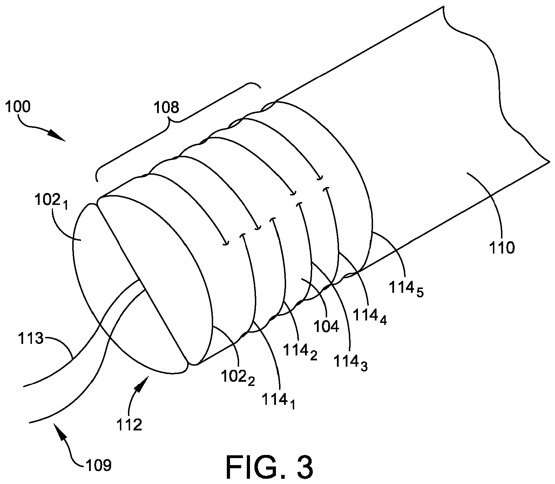

FIG. 3 illustrates a partial perspective view of one embodiment of a high-strength allograft construct 100. In this embodiment, allograft construct 100 includes first and second tendon lengths 102₁, 102₂ that have been whip stitched together at a stitched end portion 104. Stitched end portion 104 secures a common free end 112 with a whip stitched pattern 108, which, in this embodiment, may include five sutures 114₁₋₅, and an anchor 109. Stitched end portion 104 abuts an unstitched middle portion 110.

Whip stitched pattern 108 may be needle-threaded through and about first and second tendon lengths 102₁, 102₂. Pattern 108 and anchor 109 are formed of any appropriate and continuous flexible strand 113, including suture material of rope or wire that is formed of natural or manmade materials that do not react negatively with human tissue.

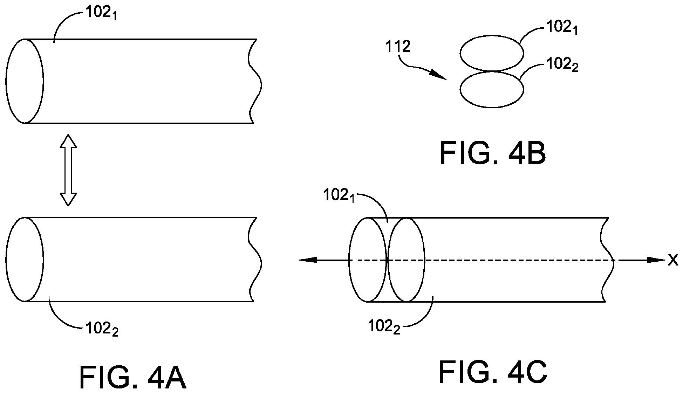

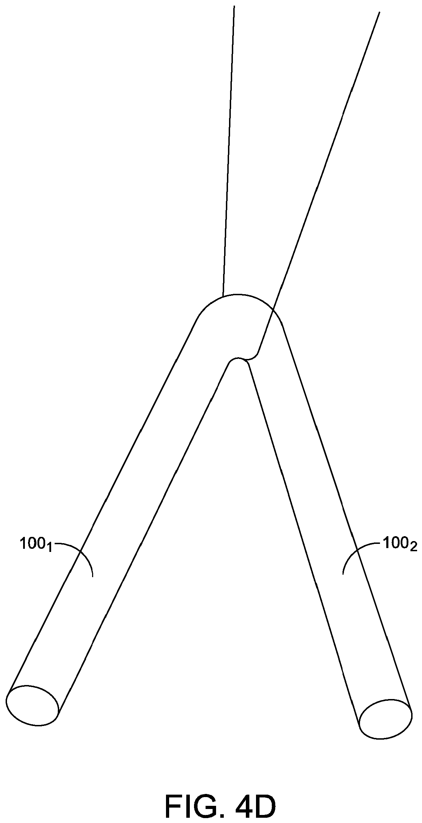

FIGS. 4-13 detail the intricacies of whip stitched pattern 108 and anchor 109 to demonstrate how high-strength allograft construct 100 resists maximum pull-out force, while also resisting suture deformation and slippage. Specifically, FIGS. 4A-4C illustrate side-partial, end, and perspective-partial views of first tendon length 102₁ and second tendon length 102₂ positioned in parallel along a longitudinal axis, X. Each of first and second tendon lengths 102₁, 102₂ may be formed of separate tendon or ligament strands. Alternatively, and as shown in FIG. 1D, first and second tendon lengths 102₁, 102₂ may be formed of first and second halves of a single tendon or ligament strand that has been folded over or doubled. In either alternative, first and second tendon lengths 102₁, 102₂ may meet at a common free end 112 (FIGS. 4B-4C).

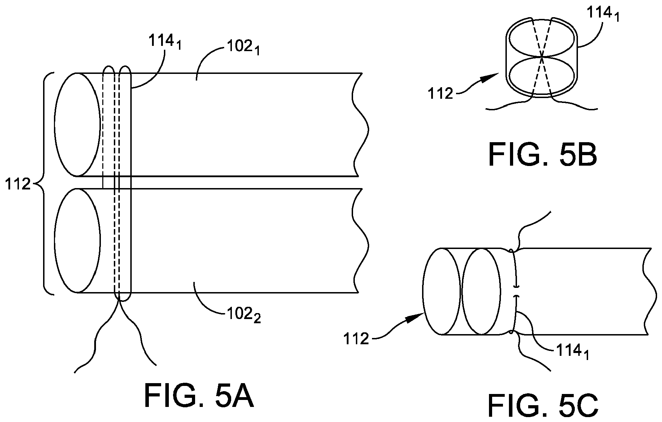

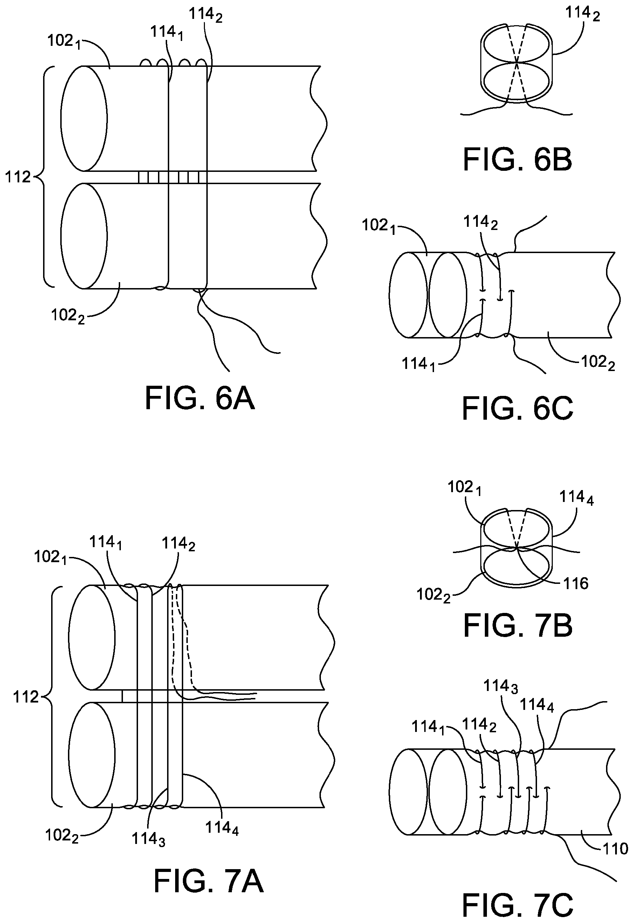

FIGS. 5A-5C and 6A-6C illustrate side-partial, end, and perspective-partial views of first and second sutures 114₁, 114₂, respectively, as applied to free end 112 of first and second tendon lengths 102₁, 102₂. As shown in FIGS. 5A-5C, first suture 114₁ threads flexible strand 113 both through and about free end 112, securing both tendon lengths 102₁, 102₂ relative to one another. Second suture 114₂, added in FIGS. 6A-6C, repeats the loop. Notably, first suture 114₁ originates at a location adjacent to free end 112. As a result, additional sutures 114₂₋ₙ progress or advance inward toward unstitched middle portion 110 (FIG. 3).

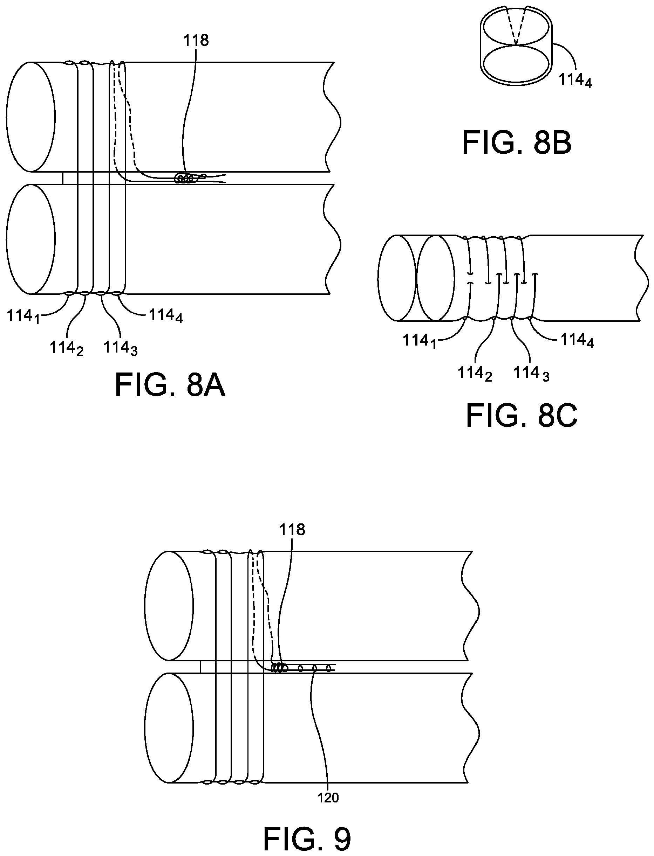

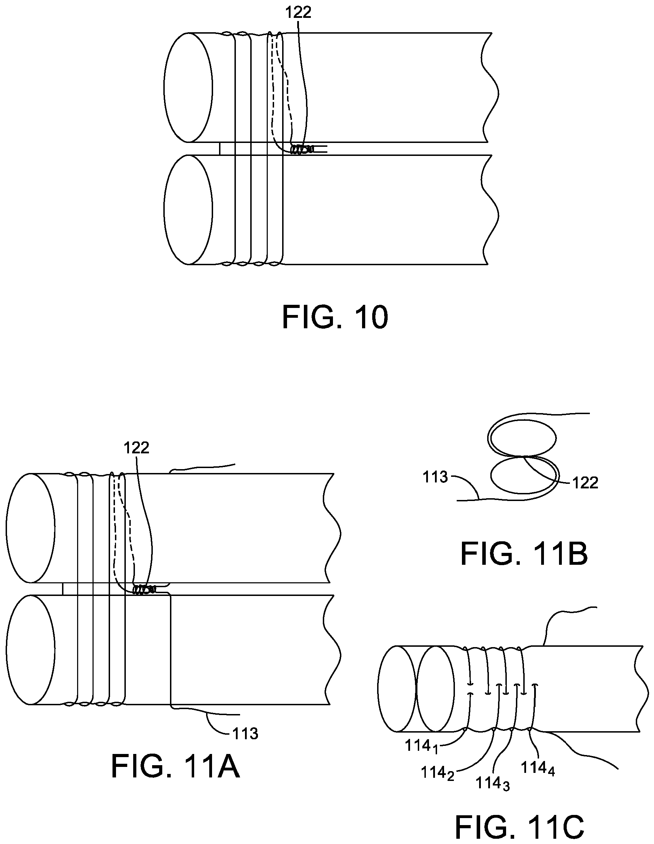

FIGS. 7A-7C illustrate side-partial, end, and perspective-partial views of first through fourth sutures 114₁₋₄, as applied to free end 112 of first and second tendon lengths 102₁, 102₂. In this embodiment, fourth suture 114₄ is the fourth of five total sutures 114₁₋₅. That is, suture 114₄ is the second-to-last suture, and, therefore, suture 114₄ may differ slightly from sutures 114₁₋₃ in that flexible strand 113 may exit the loop prior to completion at a space or junction 116 between first and second tendon lengths 102₁, 102₂, as shown in FIG. 7B. Once flexible strand 113 has exited suture 114₄, a Duncan Loop Knot 118 may be applied, as shown in FIGS. 8A-8C, and tightened. In this embodiment, Duncan Loop Knot 118 may be followed by three Reverse-Alternating-Post-Half-Hitch knots 120, shown in FIG. 9. Duncan Loop Knot 118, along with Reverse-Alternating-Post-Half-Hitch knots 120 may then be tightened to form a multiple knot bundle 122, shown in FIG. 10.

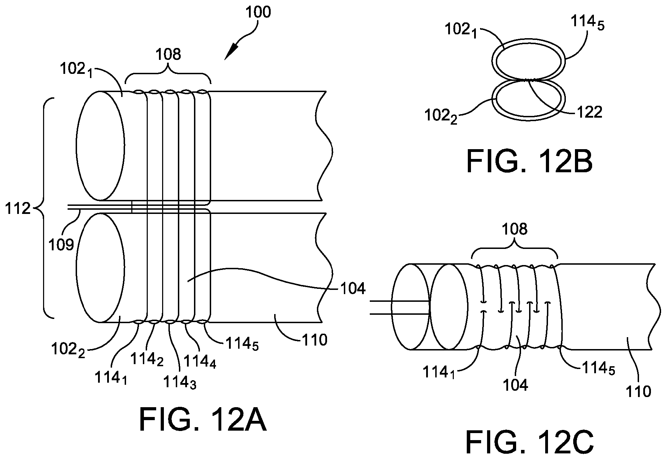

FIGS. 11A-11C and 12A-12C illustrate side-partial, end, and perspective-partial views of an initiation and completion of fifth and final suture 114₅, respectively. As shown in FIGS. 11A-11C and 12A-12C, final suture 114₅ may loop about first and second tendon lengths 102₁, 102₂ in a manner that locks-in multiple knot bundle 122, or that secures multiple knot bundle 122 beneath the flexible strand 113 of final suture 114₅. This configuration ensures that multiple knot bundle 122 is protected or safeguarded beneath the suture that experiences the highest pull-out forces during use, or beneath final suture 114₅. As force is applied, final suture 114₅ cinches about multiple knot bundle 122 and tendon lengths 102₁, 102₂, which further stabilizes multiple knot bundle 122 to resist pull-out of the knots.

The completion of final suture 114₅ completes whip stitched pattern 108. Any trailing flexible strand 113 may, in this embodiment, form anchor 109 that is strung or threaded back through the center space 116 between first and second tendon lengths 102₁, 102₂ and out free end 112 of stitched end portion 104. Anchor 109 may then be used to anchor or affix allograft construct 100 within a target bone tunnel (not shown) of a patient.

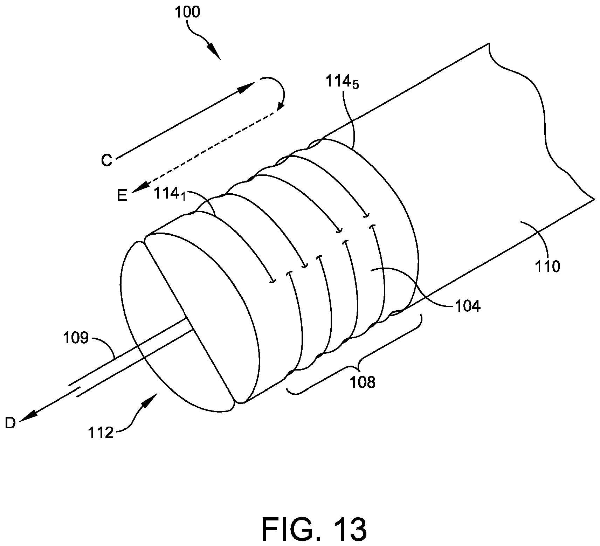

FIG. 13 illustrates a partial perspective view of completed allograft construct 100, featuring the whip stitched pattern 108. In this embodiment, as discussed above, first suture 114₁ is located adjacent to free end 112. The remaining sutures 114₂₋₅ progress inward along arrow C toward unstitched middle section 110, such that final suture 114₅ is placed at the farthest point from free end 112. As a result, tensile force applied to anchor 109 along arrow D is transferred directly to final suture 114₅ at the innermost area of stitched end 104, causing final suture 114₅ to cinch/lock about tendon lengths 102₁, 102₂ upon the application of force. Excess force is then translated to the previous suture, or fourth suture 114₄, then to suture 114s, then to suture 114₂, and finally to suture 114₁, causing whip stitched pattern 108 to tighten along arrow E like a noose when under stress.

In use, the highest force is always experienced by final suture 114₅, which is farthest from the edge of construct 100. The least amount of force is transferred to the most vulnerable first suture 114₁, located closest to the edge of construct 100. This configuration allows allograft construct 100 with whip stitched pattern 108 to resist much higher applied forces than prior art constructs (e.g., prior art construct 50 of FIG. 2) without experiencing suture pull-out and/or deformation because far more tendon material separates the most highly stressed suture from the edge of the construct.

As discussed above in the Background section, prior art whip stitched patterns typically place the first suture at an inward location toward the unstitched middle portion of the construct and progress the remaining sutures outward toward the open end/edge of the construct. As a result, the final suture in the prior art commonly resides adjacent to the free end. Thus, the highest force applied to the prior art anchor is transferred directly to the final suture, located closest to the end of the construct, which often results in suture pull-out and/or deformation.

Allograft construct 100 also serves to prevent knot pull-out because multiple knot bundle 122 (FIGS. 10 and 11A) is applied immediately prior to the final suture, or between the second-to-final suture (here, fourth suture 114₄) and the final suture (here, final suture 114₅). This positioning allows the final suture to "lock-in" the multiple knot bundle, or to tighten about the multiple knot bundle upon the application of force, thereby preventing knot pull-out.

Plane strain tensile (PST) testing was performed on six sample allograft constructs, including three prior art constructs (labeled "Old" or "O") and three constructs featuring whip stitched pattern 108 (labeled "New" or "N"). The results are shown in Table 1, below.

**Table 1: Deformation of Construct > 1 cm or Suture Failure**

| Sample | Deformation of > 1 cm | Suture yield | Whip Stitch Suture Technique |
|---|---|---|---|
| | (lbs) | (lbs) | Old OR New |
| A | na | 64.5 | N |
| B | 6.8 | na | O |
| C | na | 63.4 | N |
| D | na | 67.3 | N |
| E | 5.6 | na | O |
| F | 4.5 | na | O |

Testing criteria was set to "fail" at the point where elongation of the construct exceeded 1 cm. The force required to reach the point of failure, as well as the force at which the suture failed, were recorded to demonstrate the integrity of each suture. As shown in Table 1, none of the prior art samples maintained tissue integrity until suture yield (i.e., the construct deformed beyond 1 cm or the sutures pulled out prior to the point of suture breakage), while all of the constructs featuring stitched pattern 108 maintained tissue integrity until the suture broke at its expected value above 266.89 N (60 pounds) of force. Constructs featuring stitching pattern 108 experienced no deformation throughout the tensile strength of the suture, while all of the prior art constructs experienced deformation of the construct beyond the allowable 1 cm and did not exhibit tensile strength beyond 6.8 lbs.

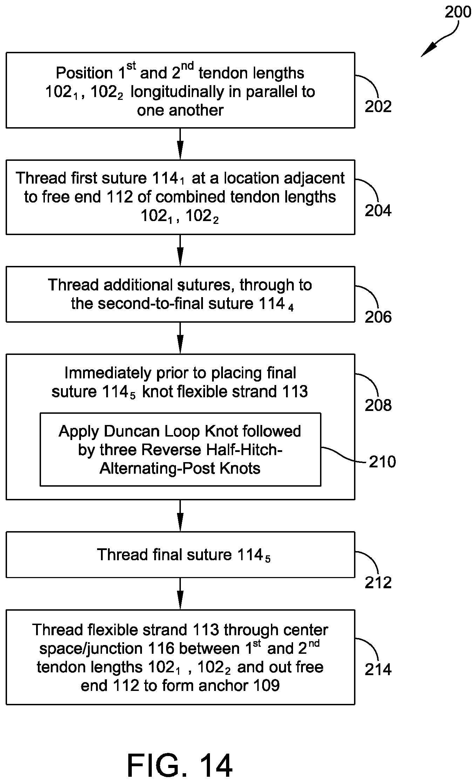

FIG. 14 provides a flow chart illustrating an exemplary method 200 of manufacturing one embodiment of high-strength allograft construct 100. Method 200 initiates with the positioning (202) of first tendon length 102₁ and second tendon length 102₂ longitudinally in parallel to one another. As discussed above, this positioning may include placing independent tendon strands side-by-side or by folding over a single tendon strand to form two distinct lengths 102₁, 102₂, as shown in FIG. 4D. Method 200 continues with the threading (204) of flexible strand 113 through and about free end 112 to form first suture 114₁ located adjacent to free end 112 of combined tendon lengths 102₁, 102₂. After the placement of first suture 114₁, method 200 may continue with the threading of additional sutures (206), such as sutures 114₂₋₄, which progress inward toward unstitched middle portion 110. Additional sutures may be threaded through to the second-to-final suture, or, in this embodiment, fourth suture 114₄. After placing the second-to-final suture and immediately prior to placing the final suture, flexible strand 113 may be knotted (208). In one embodiment, knotting flexible strand 113 (208) may involve applying a Duncan Loop Knot 118, followed by three Reverse Half-Hitch-Alternating-Post Knots 120 (210) to form multiple knot bundle 122. After knotting (208) flexible strand 113, method 200 may continue with the threading of a final suture (212) (e.g., final suture 114₅) to complete whip stitched pattern 108. Final suture 114₅ may be looped about multiple knot bundle 122 to guard against knot pull-out, as discussed above. Once the final knot has been applied (212), flexible strand 113 may be threaded through center space/junction 116 between first and second tendon lengths 102₁, 102₂ and out free end 112 to form anchor 109 (214), which may be affixed within a target bone tunnel of a patient.

While method 200 discusses whip stitched pattern as having a total of five suture, it should be understood that any appropriate number of sutures may apply.

## Claims

1. An allograft construct (100), comprising:
a first tendon length (102₁) and a second tendon length (102₂), the first and second tendon lengths (102₁, 102₂) positioned longitudinally in parallel to one another; and
a continuous flexible strand (113) forming a whip stitched pattern (108) and an anchor (109);
the whip stitched pattern (108) securing together the first and second tendon lengths (102₁, 102₂) at a free end (112), thereby forming a stitched end portion (104) that abuts an unstitched middle portion (110), **characterised in that** the whip stitched pattern (108) comprising a plurality of sutures (114) that originate adjacent to the free end (112) and progress toward the unstitched middle portion (110);
wherein the plurality of sutures (114) comprises at least a first suture (114₁) located at the free end (112) and a final suture (114₅) located adjacent to the unstitched middle portion (110); and
the anchor (109) originates at the final suture (114₅) and threads through a center space (116) between the first and second tendon lengths (102₁, 102₂) and out the free end (112) of the stitched end portion (104).

2. The allograft construct (100) of claim 1, wherein the flexible strand (113) forms a multiple knot bundle (122) immediately prior to the final suture (114₅).

3. The allograft construct (100) of claim 2, wherein the multiple knot bundle (122) comprises a Duncan-Loop knot (118) and three Reverse-Alternating-Post-Half-Hitch knots (120).

4. The allograft construct (100) of claim 2, wherein the final suture (114₅) loops about the multiple knot bundle (122) such that the final suture (114₅) locks-in the multiple knot bundle (122).

5. The allograft construct (100) of claim 1, wherein first and second halves of a folded over tendon strand form the first and second tendon lengths (102₁, 102₂), respectively.

6. A non-surgical method (200) of tendon reconstruction, comprising:
positioning a first tendon length (102₁) and a second tendon length (102₂) longitudinally in parallel to one another (202);
threading a flexible strand (113) through and about a first free end (112) of the first and second tendon lengths (102₁, 102₂) to form an allograft construct (100) having a stitched end portion (104) that abuts an unstitched middle portion (110), wherein the threading comprises forming a plurality of sutures (114) by advancing the flexible strand (113) away from a first suture (114₁) located adjacent to the first free end (112) and toward a final suture (114₅) located adjacent to the unstitched middle portion (110); and
after forming the final suture (114₅) (212), stringing the flexible strand (113) through a center space (116) between the first and second tendon lengths (102₁, 102₂) and out the first free end (112) of the stitched end portion (104).

7. The non-surgical method of claim 6, further comprising knotting the flexible strand (113) immediately prior to forming the final suture (114₅).

8. The non-surgical method of claim 7, wherein the knotting comprises applying a multiple knot bundle (122).

9. The non-surgical method of claim 8, wherein the multiple knot bundle (122) comprises a Duncan-Loop knot (118) followed by three Reverse-Alternating-Post-Half-Hitch knots (120) (210).

10. The non-surgical method of claim 8, wherein the forming of the final suture (114₅) comprises looping the flexible strand (113) about the multiple knot bundle (122) such that the final suture (114₅) locks-in the multiple knot bundle (122).

## Patentansprüche

1. Allotransplantatkonstrukt (100), umfassend:
eine erste Sehnenlänge (102₁) und eine zweite Sehnenlänge (102₂), wobei die erste und die zweite Sehnenlänge (102₁, 102₂) längs parallel zueinander positioniert sind; und
einen kontinuierlichen flexiblen Strang (113), der ein überwendlich genähtes Muster (108) und einen Anker (109) bildet;
wobei das überwendlich genähtes Muster (108) die erste und die zweite Sehnenlänge (102₁, 102₂) an einem freien Ende (112) aneinander sichert, wodurch ein genähter Endabschnitt (104) gebildet wird, der an einen nicht genähten mittleren Abschnitt (110) angrenzt, **dadurch gekennzeichnet, dass** das überwendlich genähte Muster (108) mehrere Nähte (114) umfasst, die benachbart zu dem freien Ende (112) ausgehen und sich zu dem nicht genähten mittleren Abschnitt (110) hin fortsetzt;
wobei die mehreren Nähte (114) mindestens eine erste Naht (114₁), die sich an dem freien Ende (112) befindet, und eine Endnaht (114₅), die sich benachbart zu dem nicht genähten mittleren Abschnitt (110) befindet, umfassen; und
der Anker (109) an der Endnaht (114₅) ausgeht und sich durch einen zentralen Raum (116) zwischen der ersten und der zweiten Sehnenlänge (102₁, 102₂) und aus dem freien Ende (112) des genähten Endabschnitts (104) heraus fädelt.

2. Allotransplantatkonstrukt (100) nach Anspruch 1, wobei der flexible Strang (114) ein Bündel (122) mit mehreren Knoten unmittelbar vor der Endnaht (114₅) bildet.

3. Allotransplantatkonstrukt (100) nach Anspruch 2, wobei das Bündel (122) mit mehreren Knoten einen Duncan-Loop-Knoten (118) und drei Reverse-Alternating-Post-Half-Hitch-Knoten (120) umfasst.

4. Allotransplantatkonstrukt (100) nach Anspruch 2, wobei die Endnaht (114₅) sich um das Bündel (122) mit mehreren Knoten windet, so dass die Endnaht (114₅) das Bündel (122) mit mehreren Knoten sichert.

5. Allotransplantatkonstrukt (100) nach Anspruch 1, wobei die erste und die zweite Hälfte eines umgeknickten Sehnenstrangs die erste bzw. die zweite Sehnenlänge (102₁, 102₂) bilden.

6. Nichtchirurgisches Verfahren (200) zur Sehnenrekonstruktion, umfassend:
Positionieren einer ersten Sehnenlänge (102₁) und einer zweiten Sehnenlänge (102₂), wobei die erste und die zweite Sehnenlänge (102₁, 102₂) längs parallel zueinander (202);
Fädeln eines flexiblen Strangs (113) durch und um ein erstes freies Ende (112) der ersten und der zweiten Sehnenlänge (102₁, 102₂), um ein Allotransplantatkonstrukt (100) mit einem genähten Endabschnitt (104) zu bilden, der an einen nicht genähten mittleren Abschnitt (110) angrenzt, wobei das Fädeln ein Bilden von mehreren Nähten (114) durch Vorschieben des flexiblen Strangs (113) von einer ersten Naht (114₁), die sich benachbart zu dem ersten freien Ende (112) befindet, weg und zu einer Endnaht (114₅), die sich benachbart zu dem nicht genähten mittleren Abschnitt (110) befindet, hin umfasst; und
nach dem Bilden der Endnaht (114₅) (212) Schnüren des flexiblen Strangs (113) durch einen zentralen Raum (116) zwischen der ersten und der zweiten Sehnenlänge (102₁, 102₂) und aus dem ersten freien Ende (112) des genähten Endabschnitts (104) heraus.

7. Nichtchirurgisches Verfahren nach Anspruch 6, weiterhin umfassend ein Knoten des flexiblen Strangs (113) unmittelbar vor dem Bilden der Endnaht (114₅).

8. Nichtchirurgisches Verfahren nach Anspruch 7, wobei das Knoten ein Anwenden eines Bündels (122) mit mehreren Knoten umfasst.

9. Nichtchirurgisches Verfahren nach Anspruch 8, wobei das Bündel (122) mit mehreren Knoten einen Duncan-Loop-Knoten (118), gefolgt von drei Reverse-Alternating-Post-Half-Hitch-Knoten (120) umfasst (210).

10. Nichtchirurgisches Verfahren nach Anspruch 8, wobei das Bilden der Endnaht (114₅) ein Winden des flexiblen Strangs (113) um das Bündel (122) mit mehreren Knoten, so dass die Endnaht (114₅) das Bündel (122) mit mehreren Knoten sichert, umfasst.

## Revendications

1. Construction d'allogreffe (100), comprenant :
une première longueur de tendon (102₁) et une seconde longueur de tendon (102₂), les première et seconde longueurs de tendon (102₁, 102₂) positionnées longitudinalement en parallèle les unes par rapport aux autres ; et
un fil souple continu (113) formant un motif cousu en surjet (108) et un ancrage (109) ;
le motif cousu en surjet (108) fixant ensemble les première et seconde longueurs de tendon (102₁, 102₂) à une extrémité libre (112), formant ainsi une partie d'extrémité cousue (104) qui vient en butée contre une partie médiane non cousue (110), **caractérisé en ce que** le motif cousu en surjet (108) comprenant une pluralité de sutures (114) qui sont adjacentes à l'extrémité libre (112) et progressent vers la partie médiane non cousue (110) ;
la pluralité de sutures (114) comprenant au moins une première suture (114₁) située au niveau de l'extrémité libre (112) et d'une suture finale (114₅) située de manière adjacente à la partie médiane non cousue (110) ; et
l'ancrage (109) provient au niveau de la suture finale (114₅) et s'enfile à travers un espace central (116) entre les première et seconde longueurs de tendon (102₁, 102₂) et hors de l'extrémité libre (112) de la partie d'extrémité cousue (104).

2. Construction d'allogreffe (100) selon la revendication 1, dans laquelle le brin souple (113) forme un faisceau à plusieurs noeuds (122) immédiatement avant la suture finale (114₅).

3. Construction d'allogreffe (100) selon la revendication 2, dans laquelle le faisceau à plusieurs noeuds (122) comprend un noeud d'hameçon (118) et trois noeuds à demi-attelage (120) à brins alternés inverses.

4. Construction d'allogreffe (100) selon la revendication 2, dans laquelle la suture finale (114₅) s'entoure autour du faisceau à plusieurs noeuds (122), de telle sorte que la suture finale (114₅) verrouille le faisceau à plusieurs noeuds (122).

5. Construction d'allogreffe (100) selon la revendication 1, dans laquelle des première et seconde moitiés d'un brin plié sur tendon forment les première et seconde longueurs de tendon (102₁, 102₂), respectivement.

6. Procédé non chirurgical (200) de reconstruction de tendon, comprenant :
la position d'une première longueur de tendon (102₁) et d'une seconde longueur de tendon (102₂) longitudinalement en parallèle l'un de l'autre (202) ;
le filetage d'un brin souple (113) à travers et autour d'une première extrémité libre (112) des première et seconde longueurs de tendon (102₁, 102₂) pour former une construction d'allogreffe (100) ayant une partie d'extrémité cousue (104) qui vient en butée contre une partie médiane non cousue (110), le filetage comprenant la formation d'une pluralité de sutures (114) en faisant avancer le brin souple (113) à distance d'une première suture (114₁) située de manière adjacente à la première extrémité libre (112) et vers une suture finale (114₅) située de manière adjacente à la partie médiane non cousue (110) ; et
après la formation de la suture finale (114₅) (212), l'enfilage du brin souple (113) à travers un espace central (116) entre les première et seconde longueurs de tendon (102₁, 102₂) et hors de la première extrémité libre (112) de la partie d'extrémité cousue (104).

7. Procédé non chirurgical selon la revendication 6, comprenant en outre le nouage du brin souple (113) immédiatement avant la formation de la suture finale (114₅).

8. Procédé non chirurgical selon la revendication 7, dans lequel le nouage comprend l'application d'un faisceau à plusieurs noeuds (122).

9. Procédé non chirurgical selon la revendication 8, dans lequel le faisceau à plusieurs noeuds (122) comprend un noeud d'hameçon (118) suivi de trois noeuds à demi-attelage (120) à brins alternés inverses (210).

10. Procédé non chirurgical selon la revendication 8, dans lequel la formation de la suture finale (114₅) comprend le bouclage du brin souple (113) autour du faisceau à plusieurs noeuds (122) de telle sorte que la suture finale (114₅) verrouille le faisceau à plusieurs noeuds (122).
